# FASCICULE DE BREVET EUROPEEN

(11) **EP 3 918 321 B1**
(45) Date de publication et mention de la délivrance du brevet: **05.06.2024**
(21) Numéro de dépôt: 19718773.5
(22) Date de dépôt: 29.01.2019
(51) Int. Cl.: G01N 33/24, G01N 33/00, G01N 31/12

(54) **PROCEDE ET DISPOSITIF POUR L'ANALYSE EN CONTINU DES HYDROCARBURES GAZEUX ET DU H2S DANS DES ECHANTILLONS DES BASSINS SEDIMENTAIRES**
VERFAHREN UND VORRICHTUNG ZUR KONTINUIERLICHEN ANALYSE VON GASFÖRMIGEN KOHLENWASSERSTOFFEN UND H2S IN PROBEN VON SEDIMENTÄREN BECKEN
METHOD AND DEVICE FOR THE CONTINUOUS ANALYSIS OF GASEOUS HYDROCARBONS AND H2S IN SAMPLES OF SEDIMENTARY BASINS

(43) Date de publication de la demande: 08.12.2021
(73) Titulaire: Vinci Technologies, 92022 Nanterre (FR)
(72) Inventeur: BOUTON, Nicolas, 94800 Villejuif (FR); ESPITALIE, Jean, 78110 Le Vésinet (FR)
(74) Mandataire: Alatis
(86) Numéro de dépôt international: PCT/FR2019/000014
(87) Numéro de publication internationale: WO 2020/157389

(56) Documents cités:
- EP-A1- 0 767 375
- WO-A1-2010/049609
- US-A- 4 213 763
- US-A1- 2008 026 471
- MADEC M ET AL: "Determination of organic sulphur in sedimentary rocks by pyrolysis", JOURNAL OF ANALYTICAL AND APPLIED PYROLYSIS, ELSEVIER BV, NL, vol. 8, 1 avril 1985 (1985-04-01), pages 201-219, XP026486785, ISSN: 0165-2370, DOI: 10.1016/0165-2370(85)80027-9 [extrait le 1985-04-01]

## Description

L'invention concerne un procédé et dispositif pour l'analyse en continu des hydrocarbures gazeux et du H₂S dans des échantillons de produits pétroliers.

L'invention s'intéresse plus particulièrement à un procédé pour l'analyse en continu des hydrocarbures gazeux de C1à C6 et du H₂S dans les roches sédimentaires et pour leur détermination quantitative.

En outre, le procédé et le dispositif de l'invention s'appliquent aussi à la détermination des paramètres cinétiques des hydrocarbures gazeux et du H₂S.

### ETAT DE LA TECHNIQUE ANTERIEURE

L'industrie pétrolière se tourne de plus en plus vers la production de pétroles non conventionnels en raison de l'apparition de nouvelles méthodes d'exploitation des gaz de schiste. Une bonne exploitation de ces hydrocarbures gazeux dépend de la capacité des roches explorées à libérer et à produire des quantités suffisantes de ces gaz.

Toutefois, la récupération de ces gaz devient problématique (corrosion, dangerosité vis-à-vis du personnel) dans le cas où elles contiennent des quantités importantes de H₂S.

Ainsi, il est nécessaire lors des forages exploratoires, d'une part, de caractériser les couches les plus riches d'un bassin sédimentaire et les profondeurs les plus favorables pour obtenir des quantités d'hydrocarbures suffisantes avec de faibles teneurs en H2S et, d'autre part, de distinguer les roches mères renfermant des gaz de celles qui contiennent plutôt des huiles.

Or, ces opérations se révèlent longues et difficiles par les méthodes conventionnelles de la géochimie.

En outre, en vue de la production de gaz de schiste, il est essentiel d'évaluer les risques générés par la présence de H₂S lors des forages d'exploitation.

Dans ce contexte, le FR2906482 décrit une méthode de modélisation du processus de formation du pétrole et du gaz dans les roches sédimentaires. En particulier, ce document propose un protocole expérimental visant à déterminer la cinétique de craquage thermique du kérogène et des produits pétroliers associés dans les roches mères en vue de prédire la présence et l'évaluation de la quantité de gaz produite dans une couche sédimentaire déterminée. Ce protocole comprend la pyrolyse d'échantillons pour analyser individuellement diverses classes chimiques et, notamment, celle des hydrocarbures en C1-C4 et le H₂S.

Toutefois, cette méthode s'applique sur des échantillons différents pour chaque analyse, c'est-à-dire, sur un premier échantillon pour caractériser les hydrocarbures et sur un second échantillon pour quantifier le H₂S.

Le FR2979016 décrit une méthode de prédiction de la quantité de H₂S générée par une matière carbonée soumise à un stress thermique tel qu'une pyrolyse. Toutefois, cette méthode ne cherche pas à caractériser les hydrocarbures.

Cependant, les méthodes connues ne permettent pas d'analyser conjointement les hydrocarbures gazeux et le H₂S dans un flux gazeux résultant d'une seule opération de pyrolyse à partir d'un unique échantillon de roche sédimentaire ce qui rend l'analyse et la détermination quantitative de ces composants longues et laborieuses.

### PRESENTATION DE L'INVENTION

La présente invention a pour but de résoudre les problèmes techniques posés par l'art antérieur en proposant un procédé simple et rapide permettant en peu d'étapes et à partir d'un échantillon de faible quantité et de diverses natures, d'obtenir des résultats fiables et inédits.

Ce but est atteint, selon l'invention, au moyen d'un procédé pour la l'analyse et la détermination quantitative des hydrocarbures gazeux en C₁-C₆ et du H₂S déjà présents dans les bassins sédimentaires ou pouvant être produits au cours de l'enfouissement par craquage thermique des matières organiques, ce procédé comprenant la pyrolyse sous atmosphère non oxydante d'un échantillon unique prélevé dans le bassin sédimentaire puis l'analyse des effluents gazeux de pyrolyse, caractérisé en ce qu'on entraîne les effluents gazeux de pyrolyse au moyen d'un courant de gaz inerte, on les divise en au moins deux flux distincts, le premier flux étant destiné à l'analyse des composés hydrocarbonés totaux (C1 à C40) et le second flux à l'analyse respective des hydrocarbures gazeux seuls (de C1 à C6) et du H₂S de façon à obtenir leurs profils en fonction de la température de pyrolyse. On compare ensuite lesdits profils avec des profils de référence correspondant à des teneurs connues d'hydrocarbures gazeux et de H₂S pour en déduire leurs teneurs respectives dans l'échantillon analysé.

Le procédé de la présente invention est défini dans la revendication 1.

Selon une caractéristique avantageuse du procédé de l'invention, on effectue la pyrolyse avec une vitesse de chauffe constante comprise entre 0,5°/mn et 50°/mn pour atteindre une température finale comprise entre 100°C et 950°C.

Selon une autre caractéristique, on effectue la pyrolyse sous atmosphère non oxydante au moyen d'un balayage de gaz inerte avec un débit compris entre 50 et 250 ml/mn.

De préférence, on entraîne le premier flux des effluents gazeux de pyrolyse avec un courant d'azote dont le débit est compris entre 20 et 70 ml/mn.

En outre, le procédé de l'invention prévoit d'effectuer la division des effluents de pyrolyse à une température comprise entre 400°C et 600°C.

Selon une variante spécifique du procédé, on piège les hydrocarbures lourds supérieurs à C₆ et l'eau provenant du second flux d'effluents en sortie du four de pyrolyse.

De préférence, le second flux d'effluents représente entre 20% et 50% des effluents de pyrolyse.

De manière avantageuse, on effectue l'analyse du premier flux d'effluents au moyen d'un détecteur à ionisation de flamme tandis que l'analyse du second flux d'effluents est effectuée par spectroscopie IR et/ou UV.

Le procédé de l'invention prévoit d'analyser séparément les hydrocarbures gazeux et le H₂S dans le second flux d'effluents.

Le procédé de l'invention s'applique à des échantillons choisis, notamment, dans le groupe comprenant les roches mères, les kérogènes, les charbons, les roches réservoir, les pétroles bruts et les fractions pétrolières lourdes, les résines, les asphaltènes et les prélèvements de sols.

Un autre objet de l'invention est un dispositif pour l'analyse et la quantification des hydrocarbures gazeux en C₁-C₆ et du H₂S présents dans un échantillon prélevé dans un bassin sédimentaire, ou issus de la matière organique qu'il contient quand il est soumis à un stress thermique, ledit dispositif comprenant :
- un four de pyrolyse recevant une nacelle déplacée par un piston et destinée à contenir ledit échantillon et étant pourvu d'une tubulure d'injection en gaz inerte sous pression destiné à entraîner les effluents gazeux et,
- un diviseur de flux associé à une pompe et disposé à la sortie du four pour séparer les effluents gazeux de pyrolyse en au moins deux flux distincts destinés à être dirigés, respectivement, vers au moins une première ligne d'analyse comportant un détecteur à ionisation de flamme et
- un piège à hydrocarbures supérieurs à C6 et un piège à eau disposés à la sortie du four, en aval du diviseur de flux.
Le dispositif de la présente invention est défini dans la revendication 12.

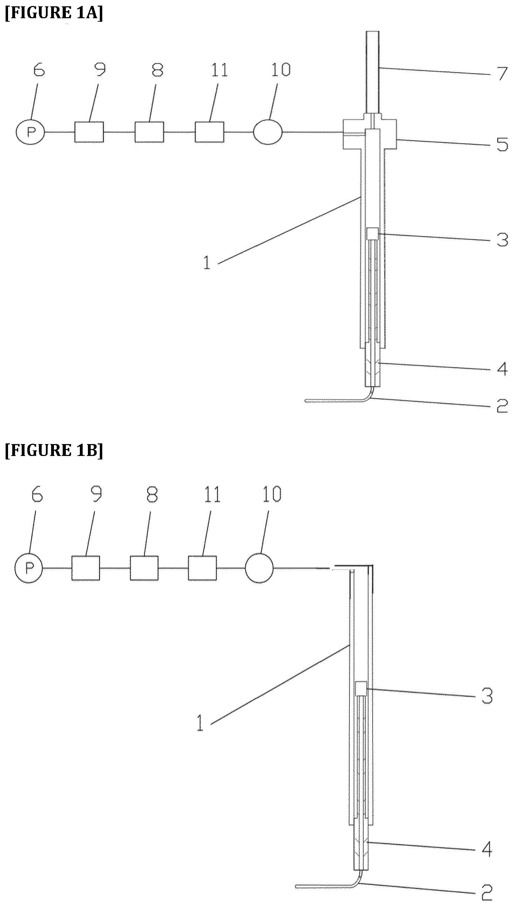

Selon une variante de réalisation, le dispositif comprend un piège à hydrocarbures supérieurs à C₆ et un piège à eau disposés à la sortie du four, en aval du diviseur de flux (cf figure 1A).

Selon une autre variante de réalisation, le dispositif comprend une seconde ligne d'analyse comportant au moins deux détecteurs à spectroscopie IR et/ou UV.

Le cas échéant, cette seconde ligne comprend, en outre, un détecteur de CO₂, de CO et/ou d'azote.

Selon encore une autre variante, le dispositif ne comporte qu'une ligne d'effluents pour l'analyse et la détermination quantitative des seuls hydrocarbures gazeux et du H₂S (figure 1B).

Le procédé et le dispositif de l'invention permettent d'analyser des échantillons de diverses natures prélevés seulement en faibles quantités au moyen de simples forages pratiqués dans les bassins sédimentaires explorés.

La mise en oeuvre du procédé de l'invention est aisée et peut être effectuée de manière totalement automatique depuis l'étape de chargement de l'échantillon dans le four de pyrolyse jusqu'à la production des résultats qui peuvent être ainsi obtenus dans des délais très courts (moins de 2 heures).

Le procédé de l'invention permet, pour chaque échantillon analysé, de calculer les distributions cinétiques des hydrocarbures totaux et des hydrocarbures gazeux qui sont calculées sur la base d'un certain nombre de réactions chimiques. Chacune de ces réactions chimiques est caractérisée par une quantité de matière organique initiale Qi, par une Energie d'Activation Ei et par une constante A appelée Constante d'Arrhénius ; la somme des Qi étant égale à la quantité totale des hydrocarbures obtenue à partir des valeurs données par le détecteur à ionisation de flamme et à la quantité totale des hydrocarbures gazeux obtenue à partir des valeurs issues de la ligne d'effluents gazeux.

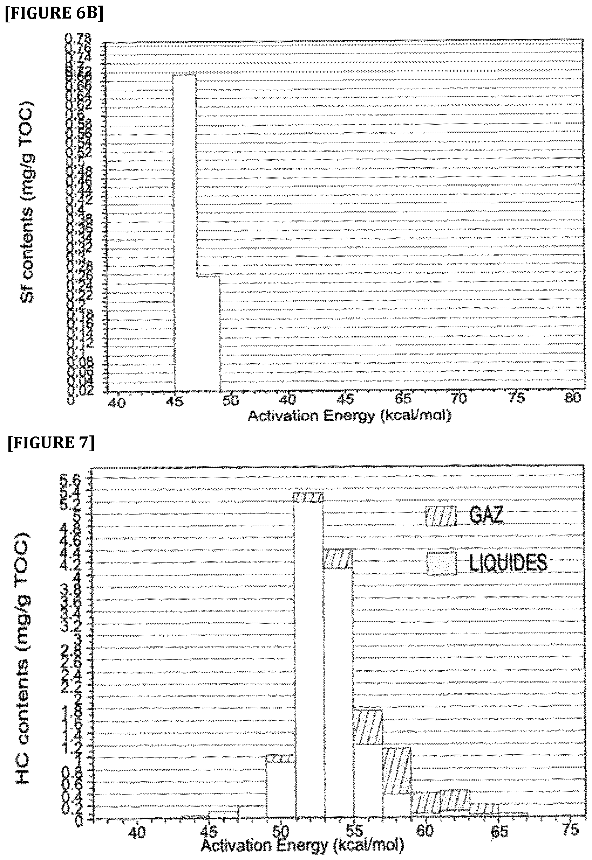

Ces distributions permettent de simuler pour chaque gaz les quantités produites en fonction du temps et de la température et de déduire des distributions cinétiques multi-compositionnelles (cf figure 7).

Ces distributions cinétiques pourront être ensuite intégrées dans des modèles mathématiques (algorithmes de logiciels) pour simuler les quantités de H₂S et de gaz que l'on peut espérer trouver dans un bassin sédimentaire.

Le procédé de l'invention peut intégrer des logiciels performants qui effectuent le tracé des courbes correspondant aux profils des hydrocarbures gazeux et du H₂S, les calculs quantitatifs, la détermination des distributions cinétiques de ces gaz et qui permettent d'obtenir des informations sur les quantités de gaz obtenues par pyrolyse, ainsi que des données inédites sur leur genèse.

### BREVE DESCRIPTION DES FIGURES

D'autres caractéristiques et avantages de l'invention ressortiront à la lecture de la description qui va suivre, en référence aux figures annexées et détaillées ci-après.
[Figure 1A] est une vue schématique d'un mode de réalisation du dispositif de l'invention.
[Figure 1B] est une vue schématique d'une variante du dispositif de l'invention qui ne comprend qu'une seule ligne d'analyse des effluents destinée à la détermination quantitative du gaz hydrocarbure et du H₂S.

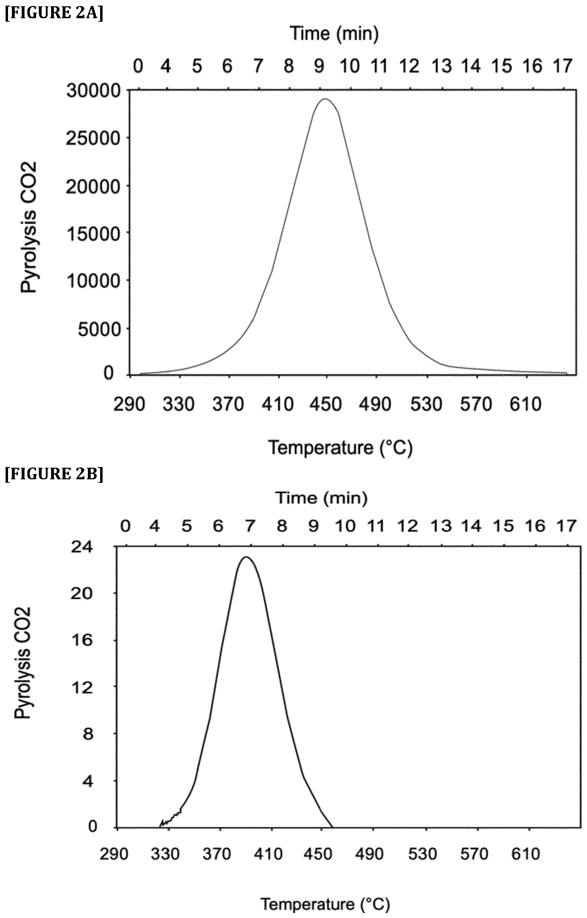

[Figure 2A] est un graphe représentant les quantités totales d'hydrocarbures gazeux dégagées au cours de l'étape de pyrolyse en fonction de la température et du temps dans le cas d'une matière organique d'origine marine.
[Figure 2B] est un graphe représentant les quantités totales de H₂S dégagées au cours de l'étape de pyrolyse en fonction de la température et du temps dans le cas d'une matière organique d'origine marine.

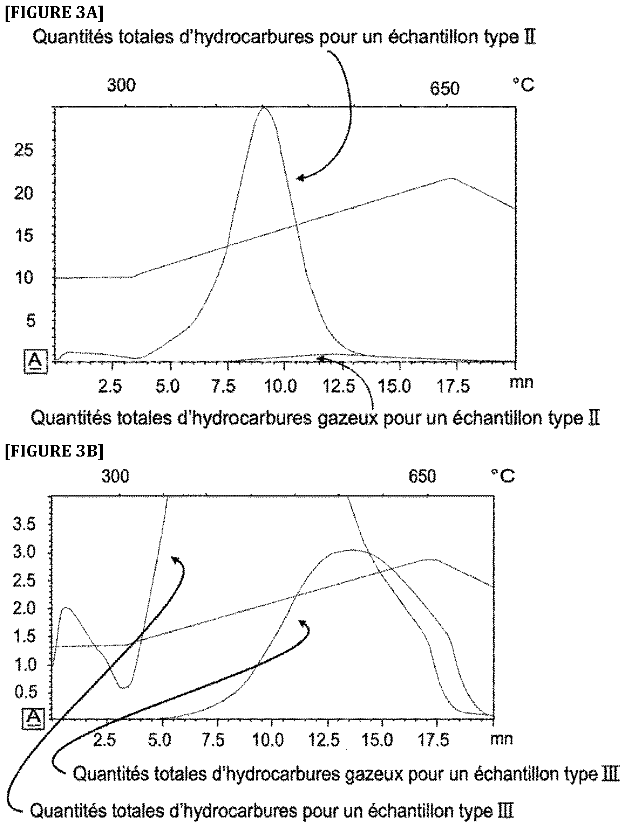

[Figure 3A] sont des graphes représentant, respectivement, les quantités totales d'hydrocarbures gazeux dégagées au cours de l'étape de pyrolyse comparées aux quantités totales des hydrocarbures produits au cours de cette pyrolyse, en fonction de la température et du temps, dans le cas d'une matière organique d'origine marine.
[Figure 3B] sont des graphes représentant, respectivement, les quantités totales d'hydrocarbures gazeux dégagées au cours de l'étape de pyrolyse comparées aux quantités totales des hydrocarbures produits au cours de cette pyrolyse, en fonction de la température et du temps, dans le cas d'une matière organique d'origine terrestre.

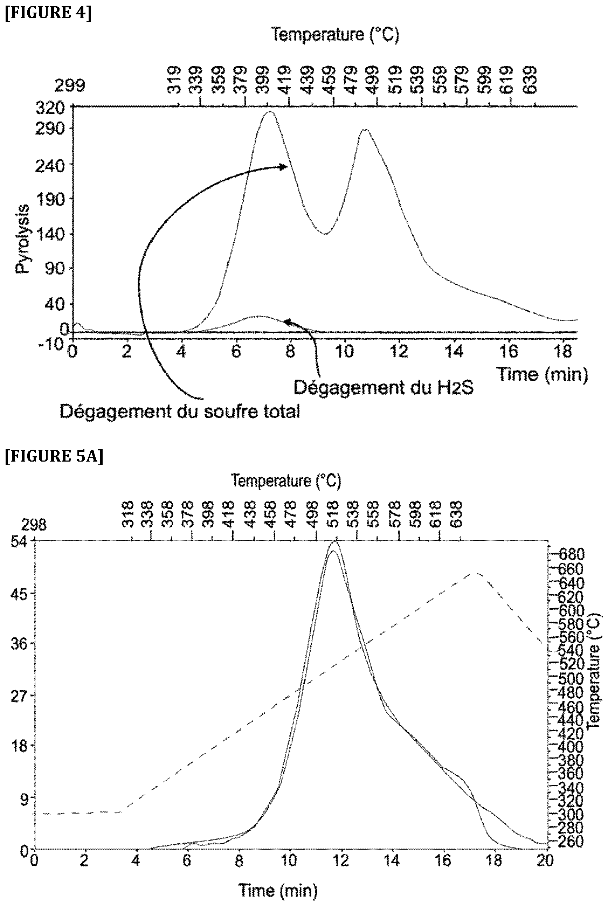

[Figure 4] sont des graphes représentant la quantité de H₂S formée comparée à la quantité totale des composés soufrés (hydrocarbures soufrés + H₂S) formés au cours de la pyrolyse.
[Figure 5A] sont des graphes représentant la production des hydrocarbures gazeux provenant du craquage thermique de la matière organique pyrolysée.

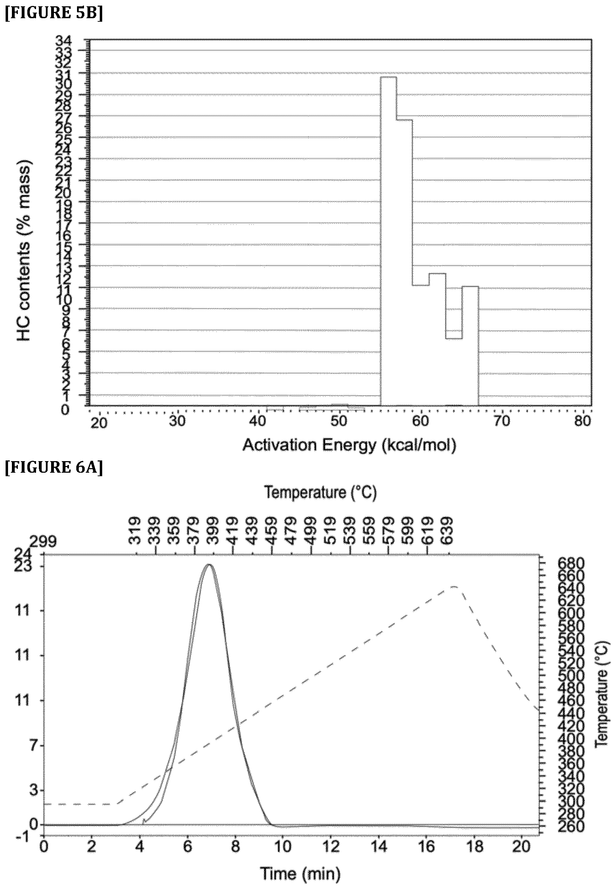

[Figure 5B] sont des graphes représentant la distribution cinétique des hydrocarbures gazeux provenant du craquage thermique de la matière organique pyrolysée.
[Figure 6A] sont des graphes représentant la production du H₂S provenant du craquage thermique de la matière organique pyrolysée dans les mêmes conditions thermiques que celles des figures 5A et 5B.
[Figure 6B] sont des graphes représentant la distribution cinétique du H₂S provenant du craquage thermique de la matière organique pyrolysée dans les mêmes conditions thermiques que celles des figures 5A et 5B.
[Figure 7] illustre un exemple de distribution cinétique compositionnelle, des gaz et liquides.

### DESCRIPTION DETAILLEE DE L'INVENTION

Naturellement, les modes de mise en oeuvre du procédé et le mode de réalisation du dispositif illustrés par les figures présentées ci-dessus et décrits ci-après ne sont donnés qu'à titre d'exemples non limitatifs. Il est explicitement prévu que l'on puisse combiner entre eux ces différents modes pour en proposer d'autres.

La figure 1A illustre un premier mode de réalisation du dispositif selon l'invention destiné à assurer l'analyse et la quantification en continu des hydrocarbures gazeux et du H₂S présent dans les roches sédimentaires et dans les produits pétroliers ainsi que la détermination des paramètres cinétiques de ces composés.

Dans cet objectif, le dispositif de l'invention comprend un four 1 destiné à la pyrolyse à une température comprise entre 50°C et 950°C, d'un échantillon unique prélevé dans le bassin sédimentaire exploré et disposé dans une nacelle 3. La pyrolyse de la matière organique produit des molécules qui vont de la plus légère (méthane) aux fractions les plus lourdes (asphaltes, par exemple).

Le four 1 peut être aussi capable d'effectuer des isothermes à des températures comprises entre 50°C et 950°C pendant des durées variant de quelques minutes à plusieurs heures.

Ce four peut être réalisé en acier inox, en alumine ou en tout autre matériau approprié. La nacelle 3 peut être réalisée en acier inox mais, dans le cas de l'analyse de composés soufrés, cette nacelle sera, de préférence, réalisée en alumine ou en porcelaine afin d'éviter la rétention du soufre sur ses parois.

La nacelle 3 est introduite de façon automatique dans le four 1 de pyrolyse au moyen d'un piston 4. Celui-ci peut être réalisé lui-aussi en acier inox mais sera de préférence réalisé en alumine ou en porcelaine pour résister à l'action du soufre.

Les moyens de chauffage du four 1 sont aptes et destinés à assurer une vitesse de chauffe constante comprise entre 0,1°/min et 50°/min pour atteindre une température finale comprise entre 50°C et 950°C.

L'étape de pyrolyse est effectuée sous atmosphère non oxydante au moyen d'un balayage de gaz inerte (par exemple de l'azote ou de l'hélium) avec un débit compris entre 50 et 250 ml/mn. Ce balayage est réalisé au moyen d'une tubulure d'injection 2 d'un gaz inerte sous pression débouchant dans le four 1.

Le courant de gaz inerte (gaz vecteur) entraîne les effluents de pyrolyse (composés organiques et minéraux générés pendant la pyrolyse) au travers du four 1 puis, en sortie de four, vers un diviseur de flux 5 dont la température est maintenue entre 400 et 600°C. Ce diviseur est, par exemple, du type de ceux décrits dans le FR2739694 et le FR2937737.

Le diviseur de flux 5 est associé à une pompe 6, à un débitmètre massique et à un jeu de vannes de régulation et permet de de diviser les effluents gazeux issus du four 1 en un premier flux représentant entre 50% et 80% des effluents et un second flux représentant la fraction complémentaire (entre 20% et 50%).

Ces flux sont entraînés et dirigés par le courant de gaz inerte, dont le débit peut être compris ente 20 et 70ml/mn, vers deux voies distinctes constituées, respectivement, d'une première ligne 51 comprenant un détecteur du type à ionisation de flamme (FID) et d'une seconde ligne 52 comprenant une série de détecteurs 8, 9 permettant d'effectuer des spectrométries UV et/ou IR.

Le FID de la première ligne détecte toutes les fractions issues de la pyrolyse alors que la seconde ligne ne détecte que les hydrocarbures gazeux (méthane, éthane, propane, butanes, pentanes...). Ainsi, par soustraction entre les résultats donnés par les deux lignes de détection, on obtient les informations sur la fraction liquide (pétrole).

Le cas échéant et comme illustré ici par le mode de réalisation de la figure 1A, le dispositif de l'invention comprend un piège 10 à hydrocarbures lourds (supérieurs à C₆) et un piège à eau 11. Ces deux pièges sont disposés à la sortie du four 1, ici en aval du diviseur de flux 5 et en amont des détecteurs 8, 9 de la seconde ligne. Le piège 11 à eau contient, par exemple, de la driérite ou du perchlorate de magnésium Mg(ClO₄)₂.

Le détecteur à ionisation de flamme 7 (FID) de la première ligne 51 mesure la totalité des hydrocarbures (C1 à C40) contenus dans les effluents de pyrolyse tandis que les détecteurs 8, 9 de la seconde ligne 52 effectuent l'analyse par spectrométrie (IR et/ou UV), respectivement, du H₂S et des hydrocarbures gazeux contenus dans ces effluents.

Selon une variante non représentée du dispositif de l'invention, il est possible de prévoir d'équiper, en outre, la seconde ligne 52 d'un détecteur de CO₂, de CO et/ou d'azote qui sont éventuellement issus de la pyrolyse.

La figure 1B représente une variante du dispositif qui ne comporte qu'une seule ligne d'analyse des effluents consacrée à la détermination quantitative du gaz hydrocarbure et du H₂S, et ne comprenant pas de ligne à détecteur du type à ionisation de flamme (FID).

Le procédé d'analyse de l'invention est effectué en plaçant dans la nacelle 3 un échantillon de sédiment géologique ou de produit pétrolier choisi, notamment, parmi les roches mères, les kérogènes, les charbons, les roches réservoir, les pétroles bruts ou les fractions de distillation ou bien les fractions pétrolières lourdes, les résines, les asphaltènes et les prélèvements de sols.

Selon la nature de l'échantillon, la masse nécessaire à l'analyse est, par exemple, la suivante :
- pour les roches (les « *cuttings* », les carottes, les prélèvements de sols) : de 5 à 100 mg
- pour les kérogènes et les charbons : 2 à 20 mg
- pour les pétroles, les huiles et les coupes de raffinage : 1 à 10 mg

A l'issue du traitement thermique par pyrolyse de l'échantillon choisi, on dispose d'un profil de H₂S et d'un profil de gaz hydrocarbure en fonction de la température de pyrolyse.

Les aires des pics de gaz hydrocarbonés et de H₂S sont comparées par étalonnage à des gaz étalons adéquats, et/ou à des aires obtenues avec des échantillons de référence dont on connaît déjà la teneur en hydrocarbures et en H₂S.

Cette approche permet de déduire les teneurs en gaz hydrocarbures et en H₂S de l'échantillon analysé. Les gaz hydrocarbures issus directement de la pyrolyse de la matière organique sont des gaz dits de craquage primaire dont les quantités viendront s'ajouter, au cours de l'enfouissement des matières organiques dans les bassins sédimentaires, aux gaz dits de craquage secondaire formés à partir des hydrocarbures déjà générés.

Par ailleurs, à partir du rapport de la quantité d'hydrocarbures gazeux sur la quantité totale d'hydrocarbures obtenue à partir de la pyrolyse d'une matière organique prélevée dans les couches peu profondes du sol, on pourra prédire si la roche qui la contient est une roche mère « dite à gaz » (cas des matières organiques d'origine continentale) ou au contraire une roche mère « dite à huile » (comme c'est le cas pour les matières organiques d'origine marine). Sa détermination est donc importante et ses caractéristiques cinétiques très utiles pour simuler la formation de l'huile et de gaz dans les bassins sédimentaires.

La présente invention permet ainsi pour chaque échantillon analysé de calculer les distributions cinétiques du gaz hydrocarbure et du H₂S directement à partir des courbes de gaz et ou de H₂S obtenues au cours de la pyrolyse Ces distributions cinétiques sont constituées par un certain nombre de réactions. Chacune de ces réactions étant caractérisée par une énergie d'activation Ei, une quantité de matière organique à l'origine Qo et une constante d'Arrhénius A, il est ainsi possible d'en déduire des distributions cinétiques multi-compositionnelles à partir des distributions cinétiques des différents hydrocarbures gazeux et des hydrocarbures totaux.

Ces distributions cinétiques pourront être ensuite intégrées dans des modèles mathématiques (algorithmes de logiciels) pour simuler et estimer les quantités d'huile et de gaz (hydrocarbures et H₂S) que l'on peut trouver dans un bassin sédimentaire.

## Revendications

1. Procédé pour la caractérisation et la quantification en continu des hydrocarbures gazeux en C₁-C₆ et du H₂S présents dans les bassins sédimentaires comprenant la pyrolyse sous atmosphère non oxydante d'un échantillon unique prélevé dans le bassin sédimentaire puis l'analyse des effluents gazeux de pyrolyse, **caractérisé en ce qu'**on entraîne les effluents gazeux de pyrolyse au moyen d'un courant de gaz inerte, on les divise en au moins deux flux distincts, le premier flux étant destiné à l'analyse des composés hydrocarbonés totaux (C1 à C40) et le second flux à l'analyse respective des hydrocarbures gazeux (C1 à C6) et du H₂S de façon à obtenir leurs profils en fonction de la température de pyrolyse puis on compare lesdits profils avec des profils de référence correspondant à des teneurs connues d'hydrocarbures gazeux et de H₂S pour en déduire leurs teneurs respectives dans l'échantillon analysé.

2. Procédé selon la revendication 1, **caractérisé en ce qu'**on effectue la pyrolyse avec une vitesse de chauffe constante comprise entre 0,5°/mn et 50°/mn pour atteindre une température finale comprise entre 100°C et 950°C.

3. Procédé selon l'une des revendications précédentes, **caractérisé en ce qu'**on effectue la pyrolyse sous atmosphère non oxydante au moyen d'un balayage de gaz inerte avec un débit compris entre 50 et 250 ml/mn

4. Procédé selon l'une des revendications précédentes, **caractérisé en ce qu'**on entraîne le premier flux des effluents gazeux de pyrolyse avec un courant de gaz inerte dont le débit est compris entre 20 et 70 ml/mn.

5. Procédé selon l'une des revendications précédentes, **caractérisé en ce qu'**on effectue la division des effluents de pyrolyse à une température comprise entre 400°C et 600°C.

6. Procédé selon l'une des revendications précédentes, **caractérisé en ce qu'**on piège les hydrocarbures lourds supérieurs à C₆ et l'eau en sortie de pyrolyse.

7. Procédé selon l'une des revendications précédentes, **caractérisé en ce que** le second flux d'effluents représente entre 20% et 50% des effluents de pyrolyse.

8. Procédé selon l'une des revendications précédentes, **caractérisé en ce qu'**on effectue l'analyse du premier flux d'effluents au moyen d'un détecteur à ionisation de flamme.

9. Procédé selon l'une des revendications précédentes, **caractérisé en ce qu'**on effectue l'analyse du second flux d'effluents par spectroscopie IR et/ou UV.

10. Procédé selon la revendication précédente, **caractérisé en ce qu'**on analyse séparément les hydrocarbures gazeux et le H₂S dans le second flux d'effluents.

11. Procédé selon l'une des revendications précédentes, **caractérisé en ce que** ledit échantillon est choisi dans le groupe comprenant les roches mères, les kérogènes, les charbons, les roches réservoir, les pétroles bruts et les fractions pétrolières lourdes, les résines, les asphaltènes et les prélèvements de sols.

12. Dispositif pour la mise en oeuvre du procédé de caractérisation et de quantification des hydrocarbures gazeux en C₁-C₆ et du H₂S selon l'une des revendications précédentes, **caractérisé en ce que** ledit dispositif comprend :
- un four (1) de pyrolyse recevant une nacelle (3) déplacée par un piston (4) et destinée à contenir ledit échantillon en étant pourvu d'une tubulure (2) d'injection en gaz inerte sous pression destiné à entraîner les effluents gazeux,
- un diviseur de flux (5) associé à une pompe (6) et disposé à la sortie du four (1) pour séparer les effluents gazeux de pyrolyse en au moins deux flux distincts destinés à être dirigés, respectivement, vers au moins une première ligne (51) d'analyse comportant un détecteur (7) à ionisation de flamme et
- un piège à hydrocarbures supérieurs à C₆ et un piège à eau disposés à la sortie du four (1), en aval du diviseur de flux (5).

13. Dispositif selon la revendication 12, **caractérisé en ce qu'**il comprend une seconde ligne (52) d'analyse comportant au moins deux détecteurs (8,9) à spectroscopie IR et/ou UV.

14. Dispositif selon la revendication précédente, **caractérisé en ce que** la seconde ligne (52) comprend, en outre, un détecteur de CO₂, de CO et/ou de N₂.

15. Dispositif selon la revendication 12, **caractérisé en ce qu'**il ne comporte qu'une ligne d'effluents pour l'analyse et la détermination quantitative des seuls hydrocarbures gazeux et du SH2

## Patentansprüche

1. Verfahren für die kontinuierliche Charakterisierung und Quantifizierung von gasförmigen C₁-C₆-Kohlenwasserstoffen und H₂S, die in Sedimentbecken vorhanden sind, umfassend die Pyrolyse unter nicht oxidierender Atmosphäre einer einzelnen Probe, die dem Sedimentbecken entnommen wird, anschließend die Analyse von Pyrolyseabgasen, **dadurch gekennzeichnet, dass** die Pyrolyseabgase mittels eines Inertgasstroms mitgeführt werden, in mindestens zwei verschiedene Strömungen geteilt werden, wobei die erste Strömung für die Analyse aller Kohlenwasserstoffverbindungen (C1 bis C40) und die zweite Strömung für die jeweilige Analyse der gasförmigen Kohlenwasserstoffe (C1 bis C6) und des H₂S bestimmt ist, so dass ihre Profile in Abhängigkeit von der Pyrolysetemperatur erhalten werden, anschließend diese Profile mit Referenzprofilen, die bekannten Gehalten an gasförmigen Kohlenwasserstoffen und H₂S entsprechen, zum Ableiten daraus ihrer jeweiligen Gehalte in der analysierten Probe verglichen werden.

2. Verfahren nach Anspruch 1, **dadurch gekennzeichnet, dass** die Pyrolyse mit einer konstanten Heizrate zwischen 0,5°/min und 50°/min zum Erreichen einer Endtemperatur zwischen 100 °C und 950 °C durchgeführt wird.

3. Verfahren nach einem der vorstehenden Ansprüche,
**dadurch gekennzeichnet, dass** die Pyrolyse unter nicht oxidierender Atmosphäre mittels einer Inertgasspülung mit einem Durchfluss zwischen 50 und 250 ml/min durchgeführt wird.

4. Verfahren nach einem der vorstehenden Ansprüche,
**dadurch gekennzeichnet, dass** die erste Strömung der Pyrolyseabgase mit einem Inertgasstrom mitgeführt wird, dessen Durchfluss zwischen 20 und 70 ml/min liegt.

5. Verfahren nach einem der vorstehenden Ansprüche,
**dadurch gekennzeichnet, dass** die Teilung der Pyrolyseabwässer bei einer Temperatur zwischen 400 °C und 600 °C durchgeführt wird.

6. Verfahren nach einem der vorstehenden Ansprüche,
**dadurch gekennzeichnet, dass** die schweren Kohlenwasserstoffe über C₆ und das Wasser an einem Pyrolyseausgang eingefangen werden.

7. Verfahren nach einem der vorstehenden Ansprüche,
**dadurch gekennzeichnet, dass** die zweite Abwasserströmung zwischen 20 % und 50 % der Pyrolyseabwässer ausmacht.

8. Verfahren nach einem der vorstehenden Ansprüche,
**dadurch gekennzeichnet, dass** die Analyse der ersten Abwasserströmung mittels eines Flammenionisationsdetektors durchgeführt wird.

9. Verfahren nach einem der vorstehenden Ansprüche,
**dadurch gekennzeichnet, dass** die Analyse der zweiten Abwasserströmung durch IR- und/oder UV-Spektroskopie durchgeführt wird.

10. Verfahren nach dem vorstehenden Anspruch,
**dadurch gekennzeichnet, dass** die gasförmigen Kohlenwasserstoffe und das H₂S in der zweiten Abwasserströmung separat analysiert werden.

11. Verfahren nach einem der vorstehenden Ansprüche,
**dadurch gekennzeichnet, dass** die Probe aus der Gruppe umfassend Muttergesteine, Kerogene, Kohlen, Speichergesteine, Rohöle und Schwerölfraktionen, Harze, Asphaltene und Bodenentnahmen ausgewählt wird.

12. Vorrichtung zum Implementieren des Verfahrens zum Charakterisieren und Quantifizieren von gasförmigen C₁-C₆-Kohlenwasserstoffen und H₂S nach einem der vorstehenden Ansprüche, **dadurch gekennzeichnet, dass** die Vorrichtung umfasst: - einen Pyrolyseofen (1), der ein Schiffchen (3) aufnimmt, das durch einen Kolben (4) bewegt wird und dazu bestimmt ist, die Probe zu enthalten, indem es mit einem Injektionsstutzen (2) für druckbeaufschlagtes Inertgas versehen ist, das dazu bestimmt ist, die Abgase mitzuführen, - einen Strömungsteiler (5), der mit einer Pumpe (6) verknüpft und an dem Ausgang des Ofens (1) angeordnet ist, zum Separieren der Pyrolyseabgase in mindestens zwei verschiedene Strömungen, die dazu bestimmt sind, jeweils zu mindestens einer ersten Analyseleitung (51) geleitet zu werden, die einen Flammenionisationsdetektor (7) aufweist, und
- eine Fangstelle für Kohlenwasserstoffe über C₆ und eine Fangstelle für Wasser, die an dem Ausgang des Ofens (1) stromabwärts des Strömungsteilers (5) angeordnet sind.

13. Vorrichtung nach Anspruch 12, **dadurch gekennzeichnet, dass** sie eine zweite Analyseleitung (52) umfasst, die mindestens zwei Detektoren (8, 9) für IR- und/oder UV-Spektroskopie aufweist.

14. Vorrichtung nach dem vorstehenden Anspruch,
**dadurch gekennzeichnet, dass** die zweite Leitung (52) ferner einen CO₂-, CO- und/oder N₂-Detektor umfasst.

15. Vorrichtung nach Anspruch 12, **dadurch gekennzeichnet, dass** sie nur eine Abwasserleitung für die Analyse und die quantitative Bestimmung allein der gasförmigen Kohlenwasserstoffe und des SH2 aufweist.

## Claims

1. Method for continuously characterizing and quantifying gaseous C₁-C₆ hydrocarbons and H₂S present in sedimentary basins, comprising pyrolysis in a non-oxidizing atmosphere of a single sample taken from the sedimentary basin, then analyzing the gaseous pyrolysis effluents, **characterized in that** the gaseous pyrolysis effluents are carried along by means of an inert gas stream, they are divided into at least two different flows, the first flow being intended for analyzing the total hydrocarbon compounds (C1 to C40) and the second flow for respectively analyzing the gaseous hydrocarbons (C1 to C6) and H₂S so as to obtain their profiles depending on the pyrolysis temperature, then said profiles are compared with reference profiles corresponding to known contents of gaseous hydrocarbons and H₂S in order to deduce therefrom their respective contents in the analyzed sample.

2. Method according to claim 1, **characterized in that** the pyrolysis is carried out at a constant heating rate of between 0.5°/mn and 50°/mn in order to achieve a final temperature of between 100°C and 950°C.

3. Method according to either of the preceding claims,
**characterized in that** the pyrolysis is carried out in a non-oxidizing atmosphere by means of inert gas flushing at a flow rate of between 50 and 250 ml/mn.

4. Method according to any of the preceding claims, **characterized in that** the first flow of the gaseous pyrolysis effluents is carried along using an inert gas stream, the flow rate of which is between 20 and 70 ml/mn.

5. Method according to any of the preceding claims, **characterized in that** the pyrolysis effluents are divided at a temperature of between 400°C and 600°C.

6. Method according to any of the preceding claims, **characterized in that** heavy hydrocarbons greater than C₆ and water are trapped at the pyrolysis outlet.

7. Method according to any of the preceding claims, **characterized in that** the second effluent flow represents between 20% and 50% of the pyrolysis effluents.

8. Method according to any of the preceding claims, **characterized in that** the first effluent flow is analyzed by means of a flame ionization detector.

9. Method according to any of the preceding claims, **characterized in that** the second effluent flow is analyzed by IR and/or UV spectroscopy.

10. Method according to the preceding claim, **characterized in that** the gaseous hydrocarbons and H₂S are analyzed separately in the second effluent flow.

11. Method according to any of the preceding claims, **characterized in that** said sample is selected from the group comprising source rocks, kerogens, coals, reservoir rocks, crude oils and heavy petroleum fractions, resins, asphaltenes and soil samples.

12. Device for implementing the method for characterizing and quantifying gaseous C₁-C₆ hydrocarbons and H₂S according to any of the preceding claims, **characterized in that** said device comprises:
- a pyrolysis furnace (1) which receives a nacelle (3) that is moved by a piston (4) and intended to contain said sample by being provided with a tube (2) for injecting pressurized inert gas intended to carry the gaseous effluents,
- a flow divider (5) associated with a pump (6) and arranged at the outlet of the furnace (1) to separate the gaseous pyrolysis effluents into at least two different flows intended to be directed, respectively, to at least one first analysis line (51) comprising a flame ionization detector (7) and
- a trap for hydrocarbons greater than C₆ and a water trap arranged at the outlet of the furnace (1), downstream of the flow divider (5).

13. Device according to claim 12, **characterized in that** it comprises a second analysis line (52) comprising at least two detectors (8, 9) for IR and/or UV spectroscopy.

14. Device according to the preceding claim, **characterized in that** the second line (52) further comprises a CO₂, CO and/or N₂ detector.

15. Device according to claim 12, **characterized in that** it comprises only one line of effluents for analyzing and quantitatively determining only gaseous hydrocarbons and SH2.
